# EUROPEAN PATENT SPECIFICATION

(11) **EP 0 381 915 B1**
(45) Date of publication and mention of the grant of the patent: **12.04.1995**
(21) Application number: 89830042.1
(22) Date of filing: 06.02.1989
(51) Int. Cl.: C07D 501/02, C07D 501/59, C07D 501/22

(54) **Process for preparing alkali metal salts of 3,7-substituted 7-aminocephalosporanic acid derivatives.**
Verfahren zur Herstellung von Alkalimetall-Salze von 3,7-substituierten 7-Aminocephalosporansauerderivaten
Procédé de préparation de sels de métaux alcalins de dérivés de l'acide 7-aminocéphalo-sporanique.

(43) Date of publication of application: 16.08.1990
(73) Proprietor: PURZER PHARMACEUTICAL CO., LTD., Taipei (TW)
(72) Inventor: Chang, S.K., Taipei Taiwan (TW); Yang, Q.C., Mason Ohio 45040 (US)
(74) Representative: Taliercio, Antonio

(56) References cited:
- FR-A- 2 124 499
- FR-A- 2 162 199
- FR-A- 2 187 303
- US-A- 3 880 842
- CHEMICAL ABSTRACTS, vol. 90, no. 2, 8th January 1979, page 643, abstract no.22940s, Columbus, Ohio, US; A. NAKOV et al.: "Synthesis of sodium salts of somealpha-aminopenicillins and alpha-aminocephalosporins", & KHIM. IND. (SOFIA) 1978, 50(7), 298-301

## Description

The present invention relates a process for the preparation of the alkali metal salts of 3, 7 - substituted 7 - aminocephalosporanic acid derivatives as antibiotics.

The derivatives of 3, 7 - substituted 7 - aminocephalosporanic acid are widely used as antibiotics to treat bacterical infections and other diseases caused by, for example staphylococci, streptococci, E. coli and various of Gram negative bacteria as well as parts of the Gram positive bacteria. Among the numerous derivatives, the most commonly used species are: 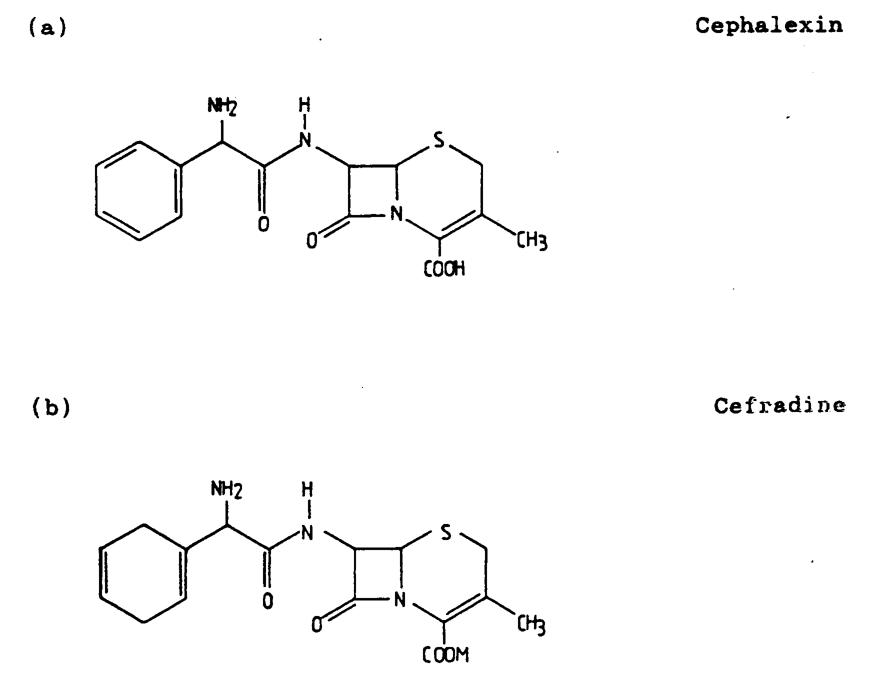 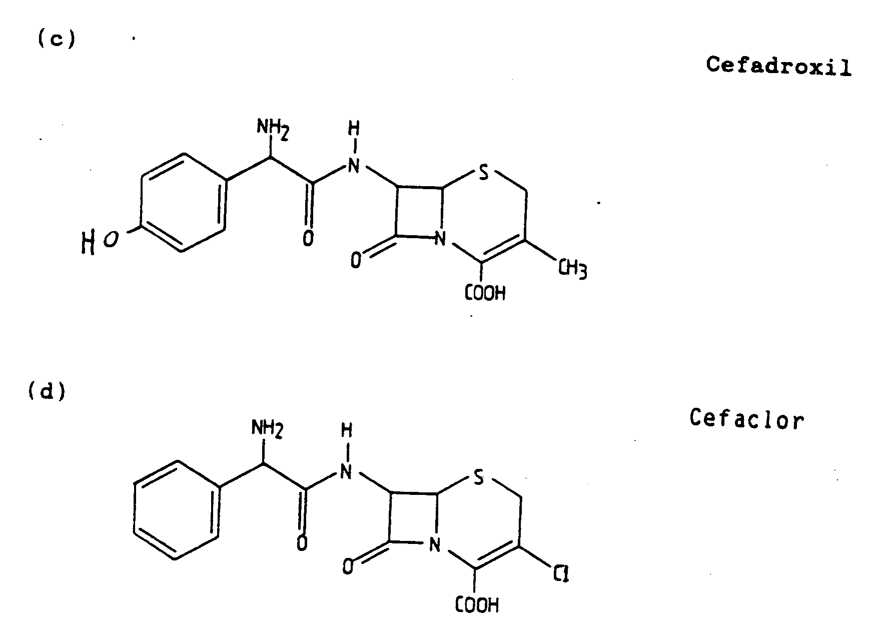
These antibiotics prove effective to many kinds of microbial infection. Unfortunately, due to their low solubility in water, these antibiotics are applied by oral administration only, and cannot be directly injected in their original (acid) form.

Injection has the fastest onset and is the only way to convey a drug to all the portions of the body. By oral administration, a gastro-intestinal absorption is required before the drug enters the blood circulation. The GI absorption also causes several restrictions of oral administration. For example, many drugs are prescribed to administer before or after the meal (and in some cases, accessory ingredients must be used) to protect their negative effects to the GI tract. Also, in oral administration, some special diets are forbidden.

Accordingly, a substitute which has the same pharmaceutical effect as the above antibiotics and which can be applied to patients by injection as well as oral administration is highly desiderable.

U.S. Patent 3,822,256 (or French Patent FR 2187303) published an antibiotics as monohydrates of sodium and potassium cephalexin which can offer a suffcient solubility.

However, this prior art does not provide a process to guarantee an ideal crystallization. An ideal crystallization is not only directly associated with high purity of the product but also greatly facilitates the separation thereof.

Moreover, the preparation in the prior art involves several compounds other than the cephalexin, including isopropyl alcohol, triethylamine, ethyl-hexanoate and acetone which have more or less toxicity to the human body. Thus, the problem of residues of these compounds in the end product cannot be neglected.

Besides the above-mentioned prior art, the U.S. Patent 3,880,842 discloses the salt of cephalosporin derivatives, in which a sodium salt can be prepared by NaHCO₃ (in water-methanol) or sodium salt of organic acids (in methanol-ethanol).

The French patent FR 2162199 discloses the derivatives of alpha-cephalosporines, which can be the sodium or potassium salt.

The French Patent FR 2124499 discloses the alkali metal salt of caphalosporin, wherein the carbonate or bicarbonate salts of alkali metal are used as basic additives.

Chemical Abstracts, vol. 90. No. 2, 8th Jan. 1979. P. 643 discloses the synthesis of sodium salts of some alphaaminocephalosporins.

Nevertheless, all these prior art references involve some compounds (as reactants or as by-products or as solvents or media) during their preparation which are more or less toxic to the human body.

Accordingly, it is an object of this invention to provide a process which ensures a perfect crystallization and involves no toxic compounds in the procedure.

This process involves the neutralization of the acid-form derivative (for example, cefradine) (represented by HD) with an alkaline base (represented by MOH):

HD + MOH MD + H₂O

Since the acid-form derivative has a low solubility in solvents, it must at first be made into a suspension, into which a saturated solution of an alkaline base (for example, NAOH) in alcohol is added and mixed to allow the neutralization to take place. Ethyl alcohol is the only ideal medium for the suspension, since it is the only organic solvent which has the least toxicity to the human body and does not cause any residual problem. Practically, a 95% alcohol is the idealest medium for the suspension. The alcohol used in the suspension and the alcohol used to dissolve the base have substantially the same water content. The water content in the alcohol may range, operably from 3 to 7%, and preferably from 4 to 6%. Beyond the operable range (greater than 7% or less than 3%) crystallization will be poor or even impossible.

Theoretically, the molar ratio of the derivative HD and the base MOH is 1 : 1, but preferably, the molar quantity of MOH slightly exceed that of HD.

The neutralization takes place under ambient temperature (20 to 30 Centigrade and preferably 20 to 25 Centigrade The practical concentration of the alcoholic suspension is 30% by weight. The reaction time is about 5 to 20 minutes. Then sow crystals of MD (for example, the sodium salt of cefradine) into the mixture and cool the temperature gradually to -40 to 12 Centigrade to allow the salt crystals to grow until the desired crystalline size is reached The salt-from derivative has a greater stability than its acid-form precursor, and therefore a longer shelf life.

### EXAMPLE 1

Prepare a 30% suspension of cefradine by adding one mole of cefradine in powder form to a 95% alcohol under room temperature. An saturated alcoholic solution containing an excess amount of NaOH (slightly more than 1 mole) is added to the suspension and well mixed. After a few minutes, add seed crystals of the sodium salt of cefradine to the mixture and cool it down to 4 Centigrade When the crystals grow to the desired size, separate them from the liquid phase by filtration.

## Claims

1. A process for preparing a salt of 3,7-substituted 7-aminocephalosporanic acid derivatives by neutralizing an alkali base MOH with a derivative in acid form having the following general formula: 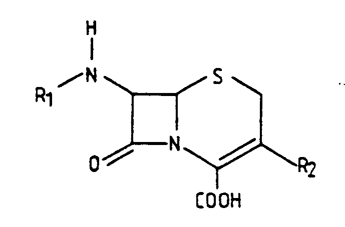 wherein R1 is an aminocarboxyl group selected from the group comprising: 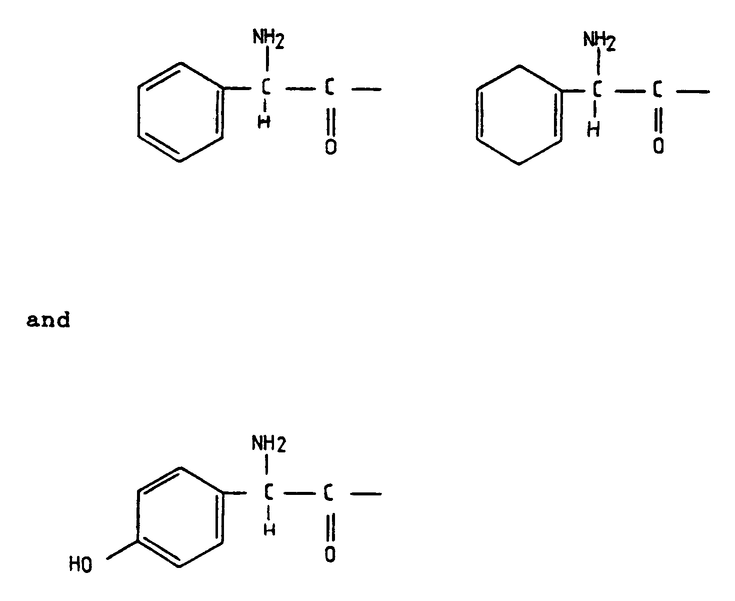 M is an alkali metal;
said process comprising:
preparing a suspension of said acid-form derivative in alcohol by mixing an acid-form derivative in powder form with an ethyl alcohol containing 3 to 7% of water under 20° to 30° Centigrade degree, and add said alkaline base in form of saturated solution in an ethyl alcohol containing 3 to 7% of water to said suspension, with the molar quantity of said base slightly exceeding that of said acid-form derivative, and allowing the resulting salt-form derivative to crystallize.

2. The process according to Claim 1, wherein the water content in said alcohol is 4 to 6%.

3. The process according to Claim 1, wherein said neutralization takes place under 20° to 25° Centigrade.

4. The process according to Claim 1, wherein the concentration of said suspension is 30% by weight.

5. The process according to Claim 1, wherein the temperature is cooled to a temperature -40° to 12° Centigrade for crystallization.

6. The process according to Claim 1, wherein the reaction time for said neutralization is 5 - 20 minutes.

## Patentansprüche

1. Verfahren zur Vorbereitung eines Salzes von Derivaten der 3,7- ersetzen 7-Aminocephalosporansaeure durch Neutralisieren einer Alkali-Base MOH mit einem Derivat in Sauereform, das die folgende allgemeine Formel hat: 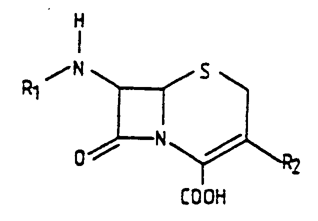 in der R₁ eine Aminocarboxyl-Gruppe ausgewaelhlt aus der Gruppe 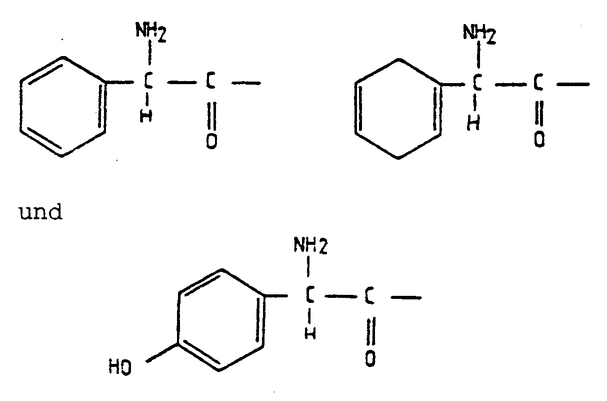 und M ein Alkalimetal ist,
welches die folgende Verfahrensschritte aufweist:
Vorbereitung einer Aufschwemmung des vorgenannten sauereformigen Derivats in Alkohol durch Zusammenmischen eines saeureformigen Derivats in Pulverform mit einem Aethylalkohol mit 3 bis 7% Wassergehalt bei einer Temperatur von 20° bis 30° und Zugabe der vorgenannten Alkali-Base in der Form einer gesaetigten Loesung in Aethylalkohol mit 3 bis 7% Wassergehalt zur vorgenannten Aufschwemmung, wobei der Molar-Anteil der vorgenannten Base hoeher ald derjenige des saeurefoermigen Derivats ist und die Krystallisierung des erhaltenen salzfoermigen Derivats gestattet.

2. Verfahren nach Anspruch 1, worin der Wassergehalt im vorgenannten Alkohol zwischen 1% und 6% liegt.

3. Verfahren nach Anspruch 1, worin die vorgenannte Neutralisation bei einer Temperatur von 20° bis 30°C stattfindet.

4. Verfahren nach Anspruch 1, worin die Konzentration der vorgenannten Aufschlemmung 30 Gew. % ist.

5. Verfahren nach Anspruch 1, worin fuer die Krystallisierung die Temperatur auf -40° bis 12°C erniedrigt wird.

6. Verfahren nach Anspruch 1, worin die Reaktion fuer die vorgenannte Neutralisation von 5 bis 20 Minuten dauert.

## Revendications

1. Procédé pour la préparation d'un sel des dérivés d'acide 7-aminocéphalosporanique 3,7-substitué par neutralisation d'une base d'alcali MOH avec un dérivé en form d'acide, ayant la forme générale suivante: 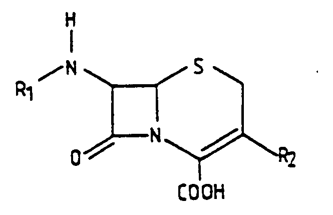 dans laquelle R₁ est un groupe aminocarboxylique choisi du groupe comprenant: 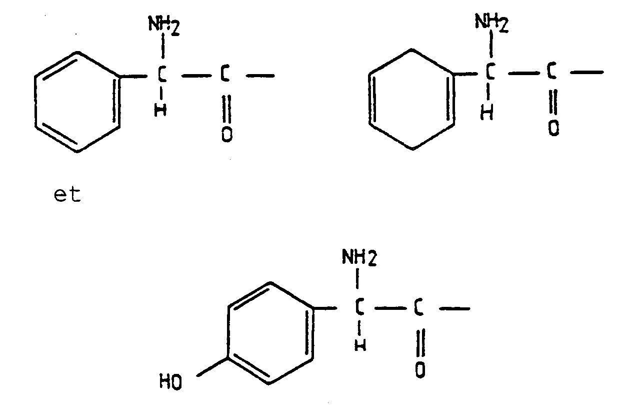 et M est un métal alcalin;
ledit procédé comprenant:
la préparation d'une suspension dudit dérivé en forme d'acide et de poudre avec un alcoole éthylique contenant de 3% à 7% d'eau à la température de 20° à 30°C et l'adjonction de ladite base d'alcali en forme d'une solution saturée dans un alcool éthylique contenant de 3% à 7% d'eau à ladite suspension, la quantité molaire de ladite base étant légerment supérieure dudit dérivé en forme d'acide et permettant la crystallisation du dérivé en forme de sel résultant.

2. Procédé selon la revendication 1, dans lequel le contenue de l'eau dans ledit alcool éthylique est de 4 à 6%.

3. Procédé selon la revendication 1, dans lequel ladite neutralisation a lieu à une température entre 20° et 25°C.

4. Procédé selon la revendication 1, dans lequel la concentration de ladite suspension est de 30% in poids.

5. Procédé selon la revndication 1, dans lequel la température est baissée pour la crystallisation entre - 40° et 12°C.

6. Procédé selon la revendication 1, dans lequel le temps de réaction de ladite neutralisation est compris entre 5 et 20 minutes.
